# EUROPEAN PATENT APPLICATION

(11) **EP 3 136 097 A1**
(43) Date of publication of application: **01.03.2017**
(21) Application number: 15782442.6
(22) Date of filing: 22.04.2015
(51) Int. Cl.: G01N 33/543, G01N 33/53, G01N 33/72

(54) **IMMUNOCHROMATOGRAPHIC ANALYSIS DEVICE, IMMUNOCHROMATOGRAPHIC ANALYSIS METHOD, AND IMMUNOCHROMATOGRAPHIC ANALYSIS KIT**

(30) Priority: 25.04.2014 JP 2014091510
(71) Applicant: Tanaka Kikinzoku Kogyo K.K., Chiyoda-ku Tokyo 100-6422 (JP)
(72) Inventor: ITO, Daisuke, Hiratsuka-shi Kanagawa 254-0076 (JP); KATO, Yuya, Hiratsuka-shi Kanagawa 254-0076 (JP); MIYATA, Aki, Hiratsuka-shi Kanagawa 254-0076 (JP)
(74) Representative: Peguet, Wilfried
(86) International application number: PCT/JP2015/062308
(87) International publication number: WO 2015/163384

(57) **Abstract**

An object is to provide an immunochromatographic analysis device capable of measuring various components contained in various analytes such as blood and urine efficiently in a short measurement time without the need for complicated measurement preparation and operations. The above object was achieved by an immunochromatographic analysis device for developing an analyte-containing solution obtained by diluting an analyte containing a detection target with an analyte dilution solution, wherein a sample addition part contains an anionic surfactant.

## Description

### TECHNICAL FIELD

The present invention relates to an immunochromatographic analysis device, an immunochromatographic analysis method, and an immunochromatographic analysis kit.

### BACKGROUND ART

Measurement of various components contained in an analyte such as blood or urine is clinically extremely important for ascertaining the physical conditions of a patient, and conventionally, various measurement methods are adopted according to the components. As one of the methods, there has been known an immunochromatographic analysis device and analysis method, in which a detection target contained in an analyte is caused to develop color by an immune reaction, and the developed color signal is confirmed.

On the other hand, there were 366 million diabetic patients in the world in 2011, and the number of diabetic patients in the world is expected to reach 552 million (about 10% of the adult population) in 2030. Further, the number of so-called prediabetic individuals is considered to be equal to or more than the number of diabetic patients.

Conventionally, the diagnosis of diabetes has been made by measuring the blood glucose level. However, recently, the concentration of glycated hemoglobin (glycohemoglobin) in which a sugar is bound to hemoglobin in the blood, particularly hemoglobin A1c (hereinafter referred to as "HbA1c") in which the N-terminal valine residue of the hemoglobin β chain has been glycated with respect to the amount of total hemoglobin reflects the average blood glucose level in the past 1 to 2 months, and therefore has begun to be used as an index suitable for diagnosis of diabetes or follow-up observation of diabetes.

The measurement of HbA1c is conventionally performed by an HPLC method, a capillary electrophoresis method, an enzymatic method, an immunological measurement method, or the like, however, these methods require expert knowledge of a specific device or analysis, and therefore have a problem that the value of HbA1c cannot be easily known in small-sized hospitals, at home, etc.

Further, with respect to the blood level of HbA1c, there are individual differences in blood components, and therefore, a positive or negative determination of diabetes is made by also simultaneously measuring hemoglobin other than HbA1c, and ascertaining the ratio of the amount of HbA1c to the amount of total hemoglobin including HbA1c and hemoglobin other than HbA1c.

However, in this case, it is necessary to separately measure HbA1c and hemoglobin other than HbA1c by the above-mentioned method, and therefore, there is also a problem that the complexity of the measurement is further increased.

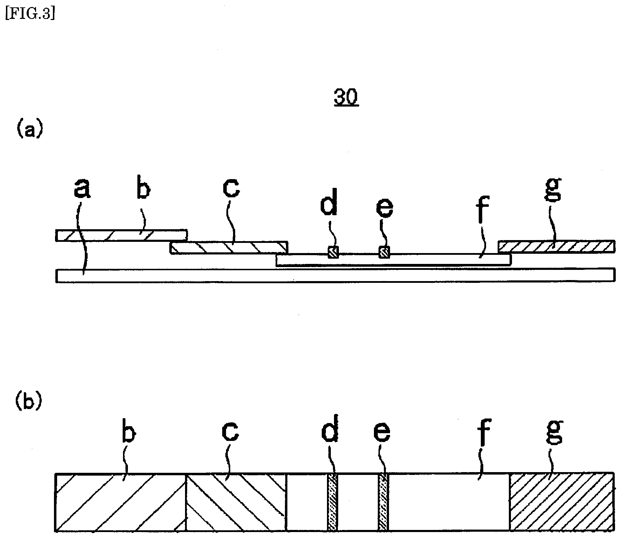

In view of this, for example, PTL 1 has proposed a method for simply measuring HbA1c. As an immunochromatographic analysis device used here, as shown in a cross-sectional view of FIG. 3(a) and a plan view of FIG. 3(b), an immunochromatographic analysis device 30 is configured such that, on a plastic adhesive sheet a, a sample pad b, a pad c containing a particle-labeled antibody labeled with a labeling substance, an antibody-immobilized membrane f, and a water absorption pad g are provided in this order along the longitudinal direction of the device, respectively, and on the antibody-immobilized membrane f, an anti-HbA1c antibody-coated part d coated with an anti-HbA1c antibody and an anti-HbA0 antibody-coated part e coated with an anti-HbA0 antibody are provided, respectively.

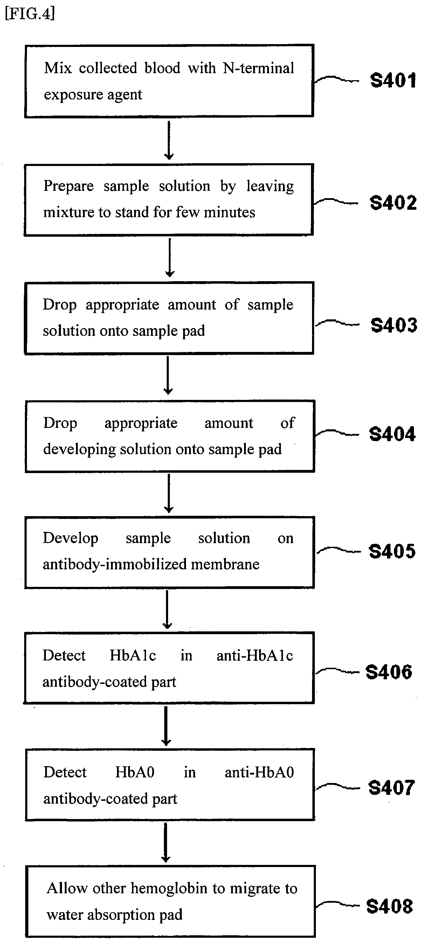

When the blood level of HbA1c is measured, as shown in the flowchart of FIG. 4, first, collected blood and a component for exposing the N-terminal of the hemoglobin β chain on the surface of a protein (N-terminal exposure agent) are mixed (S401), and the resulting mixture is left to stand for a few minutes to expose an epitope of HbA1c on the surface of a hemoglobin protein, whereby a sample solution is prepared (S402). An appropriate amount of the obtained sample solution is dropped onto a sample pad b (S403).

Subsequently, further an appropriate amount of a developing solution is dropped onto the sample pad b (S404), and the sample solution is developed on the antibody-immobilized membrane f by a capillary phenomenon (S405). The sample solution developed on the antibody-immobilized membrane f reaches the anti-HbA1c antibody-coated part d, and only HbA1c in the sample solution reacts in the part and is detected (S406).

Subsequently, the sample solution further developed on the antibody-immobilized membrane f reaches the anti-HbA0 antibody-coated part e, and only HbA0 in the sample solution reacts and is detected (S407). Other forms of hemoglobin do not react and migrate to the water absorption pad g (S408). In this manner, HbA0 and HbA1c in the sample solution are detected respectively.

### CITED REFERENCES

### PATENT DOCUMENT

Patent Document 1: JP-A-2012-251789

### DISCLOSURE OF THE INVENTION

### PROBLEMS TO BE SOLVED BY THE INVENTION

However, in the case where the above-mentioned conventional immunochromatographic analysis device is used, a step of mixing blood collected and a component for exposing the N-terminal of the hemoglobin β chain on the surface of a protein (N-terminal exposure agent) (S401), a step of preparing a sample solution by leaving the resulting mixture to stand for a few minutes (S402), a step of dropping the sample solution onto a sample pad b (S403), and a step of further preparing a developing solution separately and dropping the developing solution onto the sample pad (404) are included, and therefore, two solutions: the sample solution and the developing solution are needed, and also it is necessary to separately drop these solutions onto the sample pad b. Accordingly, it has problems that the measurement preparation and operations are complicated, and also the measurement takes time, and the efficiency is low.

In view of this, an object of the present invention is to solve the above-mentioned conventional problems and provide an immunochromatographic analysis device, an immunochromatographic analysis method, and an immunochromatographic analysis kit capable of measuring various components contained in various analytes such as blood and urine efficiently in a short measurement time without the need for complicated measurement preparation and operations.

### MEANS FOR SOLVING THE PROBLEMS

As a result of intensive studies, the present inventors found that the conventional problems as described above can be solved by incorporating an anionic surfactant in a sample addition part (sample pad) to which an analyte containing a detection target is added, and thus, the present invention could be completed.

That is, the present invention is as follows.
1. An immunochromatographic analysis device for developing an analyte-containing solution obtained by diluting an analyte containing a detection target with an analyte dilution solution, comprising a sample addition part, a labeling substance retaining part, a chromatography medium part having a detection part supported thereon, and an absorption part, wherein
   the sample addition part contains an anionic surfactant.
2. The immunochromatographic analysis device as described in 1 above, wherein the sample addition part is one member selected from the group consisting of glass fiber, cellulose, and polyethylene terephthalate.
3. The immunochromatographic analysis device as described in 1 or 2 above, wherein the sample addition part contains the anionic surfactant in an amount of 8 to 800 µg.
4. The immunochromatographic analysis device as described in any one of 1 to 3 above, wherein the detection target is a glycated protein in blood.
5. The immunochromatographic analysis device as described in 4 above, wherein the glycated protein in blood is HbA1c.
6. The immunochromatographic analysis device as described in any one of 1 to 5 above, wherein the anionic surfactant is sodium dodecyl sulfate (SDS).
7. An immunochromatographic analysis method for detecting a detection target contained in an analyte using an immunochromatographic analysis device which includes a sample addition part containing an anionic surfactant, a labeling substance retaining part, a chromatography medium part having a detection part supported thereon, and an absorption part, comprising the following steps (1) to (4):
   (1) a step of adding an analyte-containing solution obtained by diluting an analyte with an analyte dilution solution to the sample addition part;
   (2) a step of recognizing the detection target by a labeling substance retained in the labeling substance retaining part;
   (3) a step of developing the analyte and the labeling substance in the chromatography medium part as a mobile phase; and
   (4) a step of detecting the detection target in the developed mobile phase in the detection part.
8. The method as described in 7 above, wherein the analyte dilution solution contains a nonionic surfactant.
9. The method as described in 8 above, wherein the analyte dilution solution contains the nonionic surfactant in an amount of 0.01 to 5 mass%.
10. The method as described in any one of 7 to 9 above, wherein the sample addition part contains the anionic surfactant in an amount of 8 to 800 µg.
11. An immunochromatographic analysis kit, comprising an immunochromatographic analysis device, which includes a sample addition part, a labeling substance retaining part, a chromatography medium part having a detection part supported thereon, and an absorption part, and an analyte dilution solution for diluting and developing an analyte, wherein the analyte dilution solution contains a nonionic surfactant, and the sample addition part contains an anionic surfactant.

### EFFECT OF THE INVENTION

The immunochromatographic analysis device, the immunochromatographic analysis method, and the immunochromatographic analysis kit of the present invention are configured such that a sample addition part contains an anionic surfactant, and therefore, unlike the conventional art, two solutions: a sample solution and a developing solution are not needed, and it is only necessary to drop one solution: an analyte-containing solution obtained by diluting an analyte containing a detection target with an analyte dilution solution onto the sample addition part.

That is, in the present invention, an anionic surfactant is incorporated in the sample addition part, and therefore, it is not necessary to include a step of exposing an epitope of a detection target before adding an analyte-containing solution to the sample addition part. Therefore, according to the present invention, various components contained in various analytes such as blood and urine can be measured efficiently in a short measurement time without the need for complicated measurement preparation and operations.

### BRIEF DESCRIPTION OF DRAWINGS

[FIG. 1] FIG. 1(a) and FIG. 1(b) are schematic views for explaining one example of an immunochromatographic analysis device of the present invention, and FIG. 1 (a) is a cross-sectional view, and FIG. 1(b) is a plan view.
[FIG. 2] FIG. 2 is a flowchart when measuring the concentration of HbA1c with respect to the amount of total hemoglobin in blood using an immunochromatographic analysis kit of the present invention.
[FIG. 3] FIG. 3(a) and FIG. 3(b) are schematic views for explaining an immunochromatographic analysis device of a conventional art, and FIG. 3(a) is a cross-sectional view, and FIG. 3(b) is a plan view.
[FIG. 4] FIG. 4 is a flowchart when measuring the concentration of HbA1c with respect to the amount of total hemoglobin in blood using an immunochromatographic analysis device of a conventional art.

### EMBODIMENTS FOR CARRYING OUT THE INVENTION

Hereinafter, the present invention will be described in more detail.

Examples of the analyte to be used in the present invention include blood samples such as blood, plasma, and serum, urine, saliva, spinal fluid, sweat, tear, amniotic fluid, nipple discharge fluid, nasal discharge, sputum, a nasal swab, a pharyngeal swab, exudate from the skin, and extracts from tissues, cells, and feces.

Examples of the detection target in the present invention include tumor marker substances such as α-fetoprotein (AFP) and carcinoembryonic antigens (CEA), serum proteins such as ferritin, prostate-specific antigens (PSA), and immunoglobulin G (IgG), hormone-related substances such as rheumatoid factors, growth hormone (GH), and adrenocorticotropic hormone (ACTH), influenza viruses, adenoviruses, bacterial antigens such as Chlamydia antigen and Streptococcus pyogenes antigen, and hemoglobin-derived proteins.

Examples of the detection target in the present invention, preferably, a detection target in blood, include hemoglobin-derived proteins, plasma proteins, lipoproteins, secretory proteins, hormones, complements, lipids, cholesterol, sugars, other components in the body, drugs administered to the body, and metabolites thereof.

Above all, from the viewpoint of the effect of the present invention, the detection target is preferably glycated proteins such as glycated albumin and glycated hemoglobin in blood, more preferably HbA1c. Hereinafter, a description will be made by showing an example in which HbA1c is used as a detection target, and hemoglobin other than HbA1c is used as a substance to serve as a standard for making a positive or negative determination, however, the present invention is not limited to the following example.

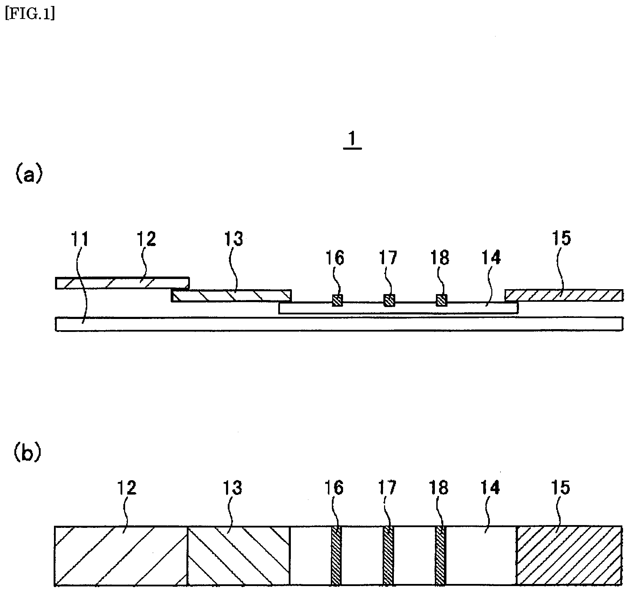

The immunochromatographic analysis device of the present invention includes a sample addition part, a labeling substance retaining part, a chromatography medium part having a detection part supported thereon, and an absorption part. Hereinafter, the chromatographic analysis device of the present invention will be described with reference to the drawings. FIG. 1(a) and FIG. 1(b) are schematic views for explaining one example of the immunochromatographic analysis device of the present invention, and FIG. 1(a) is a cross-sectional view, and FIG. 1 (b) is a plan view.

As shown in FIG. 1(a) and FIG. 1(b), an immunochromatographic analysis device 1 includes a sample addition part 12, a labeling substance retaining part 13 containing an anti-hemoglobin antibody labeled with a labeling substance, a chromatography medium part 14, and an absorption part 15, which are provided on a plastic adhesive sheet 11 in this order along the longitudinal direction of the kit, respectively.

Further, on the chromatography medium part 14, as detection parts, an anti-HbA1c antibody-coated part 16 coated with an anti-HbA1c antibody, an anti-hemoglobin antibody-coated part 17 coated with an anti-hemoglobin antibody, and an anti-IgG antibody-coated part 18 coated with an anti-IgG antibody as a control are provided respectively.

The plastic adhesive sheet 11 serves as a base material of the immunochromatographic analysis device 1, and one surface is made to serve as an adhesive surface by coating one surface with an adhesive or by sticking an adhesive tape to one surface, and part or the entire of the respective constituent parts described below are closely adhered onto the adhesive surface. As a material of the plastic adhesive sheet 11, a material which is impermeable to a sample and also is impermeable to moisture may be appropriately selected.

The sample addition part 12 can be composed of a porous sheet having properties such that it quickly absorbs the below-mentioned analyte-containing solution, but has a low ability to retain the analyte-containing solution so that the analyte-containing solution quickly migrates to a region where an antigen-antibody reaction occurs.

Examples of the porous sheet include a pad, a fiber, a membrane, etc. composed of glass fiber, cellulose, polyethylene terephthalate, polyurethane, polyacetate, cellulose acetate, nitrocellulose, nylon, a cotton cloth, etc. Above all, the sample addition part 12 is preferably formed using one member selected from the group consisting of glass fiber, cellulose, and polyethylene terephthalate.

The sample addition part 12 contains an anionic surfactant. Examples of the anionic surfactant include alkyl sulfates such as sodium dodecyl sulfate (SDS), polyoxyethylene alkyl ether sulfates such as sodium polyoxyethylene lauryl ether sulfate, alkyl benzene sulfonates such as sodium dodecyl benzene sulfonate, acylamino acid salts such as lauroyl methyl alanine and sodium N-lauroyl sarcosine, sodium dialkyl sulfosuccinate, a sodium salt of β-naphthalene sulfonate formalin condensate, and a special polycarboxylic acid-based polymer surfactant.

Among the anionic surfactants, alkyl sulfates such as sodium dodecyl sulfate (SDS) are particularly preferred from the viewpoint of improving the effect of the present invention.

In the sample addition part 12, the anionic surfactant can be contained in an amount of, for example, 8 to 800 µg, and is contained in an amount of preferably 10 to 500 µg, more preferably 16 to 480 µg. Further, in the sample addition part 12, the anionic surfactant can be contained in an amount of 5 to 500 µg/cm², and is contained in an amount of preferably 6 to 313 µg/cm², more preferably 10 to 300 µg/cm².

In order to allow the sample addition part 12 to retain the anionic surfactant in a dry state, a means such as freeze-drying, hot-air drying, natural drying, or the like may be appropriately adopted.

The anionic surfactant has a function to expose an epitope of HbA1c in collected blood on the surface of a hemoglobin protein. Specifically, the anionic surfactant functions as a component for exposing the N-terminal of the hemoglobin β chain on the surface of a protein (N-terminal exposure agent).

In the present invention, the anionic surfactant is incorporated in the sample addition part 12, and therefore, it is not necessary to include a step of exposing an epitope of a detection target before adding the below-mentioned analyte-containing solution to the sample addition part.

At this time, when one member selected from the group consisting of glass fiber, cellulose, and polyethylene terephthalate is used as a material forming the sample addition part 12, the contact efficiency between HbA1c and the anionic surfactant becomes high, and thus, an effect that the anionic surfactant efficiently acts as the N-terminal exposure agent is exhibited.

In addition, it is also possible to add any of a variety of additives such as sodium thiocyanate, guanidine hydrochloride, and EDTA to the sample addition part 12 as needed.

The labeling substance retaining part 13 retains, for example, an anti-hemoglobin antibody labeled with a labeling substance. The anti-hemoglobin antibody in the labeling substance retaining part 13 can specifically bind to hemoglobin in an analyte-containing solution. Examples of such an antibody include a polyclonal antibody and a monoclonal antibody. The monoclonal antibody and the polyclonal antibody or fragments thereof are known and available, and can be prepared by a known method.

Examples of an animal species that produces the antibody include a human being, a mouse, a rat, a rabbit, and a goat. The immunoglobulin may be any of IgG, IgM, IgA, IgE, and IgD.

The monoclonal antibody is obtained according to a conventional method as follows. The spleen cells and myeloma cells of a mouse immunized with an antigen are fused, and a hybridoma which produces a desired antibody is selected, and a monoclonal antibody produced from this hybridoma is obtained (see, for example, the method of Kohler and Milstein [Nature, 256 (1975), 495-497]).

The polyclonal antibody is obtained by separating a desired antibody from an antiserum obtained by immunizing an antibody-producing animal (for example, a human being, a mouse, a rat, a rabbit, a goat, a horse, etc.) with an antigen according to a conventional method.

As the labeling substance, a colored substance capable of visually confirming coloration is preferred, and a substance known in the art can be appropriately adopted. Examples thereof include colloidal metal particles, colloidal non-metal particles, colored latex, and an enzyme label, however, colloidal metal particles whose color hardly fades even if time has passed are particularly preferred from the viewpoint of stability of the label.

Examples of the colloidal metal particles include colloidal gold, platinum, copper, silver, and palladium, and other than these, particles obtained by mixing these colloidal metals. In particular, colloidal gold particles are preferred from the viewpoint that the particles having an appropriate particle diameter exhibit red color.

The average particle diameter of the colloidal metal particles is in the range of, for example, 1 to 500 nm, from the viewpoint of obtaining a strong color tone, preferably 10 nm to 150 nm, more preferably 20 to 100 nm. As the colloidal non-metal particles, colloidal selenium and the like can be exemplified. The colloidal metal particles and the colloidal non-metal particles can be prepared according to a conventional method, and at this time, the particle diameter is adjusted so as to exhibit a desired color tone. In addition, it is also possible to use a commercially available product.

Examples of the colored latex include high-molecular weight polymer particles such as polystyrene particles colored with a coloring agent exhibiting red or blue color, and such colored latex can be prepared according to a conventional method. Examples of the enzyme label include peroxidase, alkaline phosphatase, glucose oxidase, and galactosidase. In the case where the enzyme label is used, a substrate for the enzyme and, according to need, a color-developing reagent are allowed to act on the enzyme, and the color developed by the reaction is detected.

Incidentally, the preparation of the antibody labeled with a labeling substance can be performed according to a known method. For example, as a method for supporting colloidal gold particles on the antibody, a known method such as physical adsorption or chemical binding can be employed. Specifically, for example, the antibody is added to a solution in which gold particles are colloidally dispersed to cause a physical adsorption, and thereafter, a blocking protein such as a bovine serum albumin solution is added thereto to block the surface of the particle to which the antibody is not bound, whereby the preparation can be performed. In addition, for the antigen-antibody reaction, a known sandwich method, a competition method, a method combining these methods can be adopted.

Examples of a material of the labeling substance retaining part 13 include glass fiber, cellulose, polyethylene terephthalate, polyurethane, polyacetate, nylon, and a cotton cloth.

The chromatography medium part 14 may be any as long as it can absorb a sample analyte by a capillary phenomenon and allows the sample analyte to migrate therein, and can be composed of, for example, nitrocellulose, cellulose acetate, nylon, polyether sulfone, polyvinyl alcohol, polyester, glass fiber, polyolefin, cellulose, a mixed fiber thereof, or the like.

The anti-HbA1c antibody-coated part 16, the anti-hemoglobin antibody-coated part 17, and the anti-IgG antibody-coated part 18 provided on the chromatography medium part 14 are composed of, for example, a material capable of supporting and fixing each antibody, and examples of the material include nitrocellulose.

Examples of a material of the absorption part 15 include a material having an ability to quickly absorb an excess amount of an analyte-containing solution, and cellulose fiber, a glass filter paper, or the like is used.

Next, the immunochromatographic analysis method of the present invention will be described. The immunochromatographic analysis method of the present invention is a method for detecting a detection target contained in an analyte using the immunochromatographic analysis device described above, and includes the following steps (1) to (4):
(1) a step of adding an analyte-containing solution obtained by diluting an analyte with an analyte dilution solution to the sample addition part;
(2) a step of recognizing the detection target by a labeling substance retained in the labeling substance retaining part;
(3) a step of developing the analyte and the labeling substance in the chromatography medium part as a mobile phase; and
(4) a step of detecting the detection target in the developed mobile phase in the detection part.

Hereinafter, the respective steps will be described.

In the step (1), first, an analyte-containing solution is prepared by diluting an analyte with an analyte dilution solution. The analyte dilution solution can act as a developing solution for developing an analyte and a labeling substance in the chromatography medium part as a mobile phase in the step (3). The analyte dilution solution preferably contains a nonionic surfactant from the viewpoint that it has favorable developing properties and also does not inhibit the antigen-antibody reaction.

Examples of the nonionic surfactant include polyoxyethylene alkyl ethers, polyoxyethylene/polyoxypropylene alkyl ethers, polyoxyethylene sorbitan fatty acid esters, polyoxyethylene p-t-octylphenyl ethers, polyoxyethylene p-t-nonylphenyl ethers, alkyl polyglucosides, fatty acid diethanolamide, and alkyl monoglyceryl ethers.

In the analyte dilution solution, for example, water is used as a solvent, and the nonionic surfactant can be contained in a proportion of, for example, 0.01 to 5 mass%, and is contained preferably in a proportion of 0.03 to 3.0 mass%, more preferably in a proportion of 0.3 to 3.0 mass%. Further, in order to adjust the pH, an additive such as a buffer or an inorganic salt may be added as needed.

From the viewpoint of improving the effect of the present invention, it is preferred that the ratio of the anionic surfactant contained in the sample addition part 12 to the nonionic surfactant contained in the analyte dilution solution is set to a given value, and specifically, the ratio of the anionic surfactant to the nonionic surfactant (mass ratio) is preferably from 9:1 to 1:90, more preferably from 3:1 to 1:60, further more preferably from 1:2 to 1:50, and most preferably from 1:2 to 1:34.

The thus obtained analyte dilution solution is mixed with blood collected from a patient, whereby an analyte-containing solution is prepared and dropped onto the sample addition part 12. Examples of a site where blood is collected from a patient include a finger, a gum, an arm vein, and an ear.

HbA1c contained in the analyte-containing solution reaches the labeling substance retaining part 13 by a capillary phenomenon while exposing the N-terminal of the hemoglobin β chain on the surface of a protein by the anionic surfactant contained in the sample addition part 12.

In the step (2), hemoglobin in the analyte-containing solution having reached the labeling substance retaining part 13 is subjected to an antigen-antibody reaction with the anti-hemoglobin antibody labeled with a labeling substance, whereby a complex is formed. That is, HbA1c, which is the detection target, is also recognized by the labeling substance retained in the labeling substance retaining part 13.

In the step (3), the analyte and the labeling substance, that is, the analyte-containing solution and the complex of the anti-hemoglobin antibody and hemoglobin are developed in the chromatography medium part 14 as a mobile phase. At this time, the nonionic surfactant contained in the analyte-containing solution exhibits an effect that the developing properties in the development are improved and also the subsequent antigen-antibody reaction is not inhibited as described above.

Subsequently, in the step (4), HbA1c, which is the detection target in the mobile phase developed in the chromatography medium part 14 reaches the anti-HbA1c antibody-coated part 16, and HbA1c reacts with the anti-HbA1c antibody while it is passing through the part and is immobilized thereon. Hemoglobin other than HbA1c and the analyte-containing solution do not react and pass through the anti-HbA1c antibody-coated part 16, however, when reaching the anti-hemoglobin antibody-coated part 17, hemoglobin other than HbA1c reacts with the anti-hemoglobin antibody and is immobilized thereon.

Incidentally, the labeling substance which does not react with the antigen or the labeling substance which does not react in the antibody-coated parts 16 and 17 react with the anti-IgG antibody in the anti-IgG antibody-coated part 18 and is immobilized thereon, and develops color as a control showing that the development is performed normally.

The other components of the sample solution do not react and migrate to the absorption part 15. In this manner, the developed color signals due to the presence of HbA1c and hemoglobin other than HbA1c can be confirmed in the respective coated parts. Incidentally, the anti-HbA1c antibody and the anti-hemoglobin antibody may be either a monoclonal antibody or a polyclonal antibody as described above.

There are individual differences in blood components, and it is difficult to make the determination of diabetes only by causing HbA1c to develop color and confirming the strength of the developed color. Therefore, it is preferred that HbA1c and hemoglobin other than HbA1c are caused to simultaneously develop color and the determination is made by comparison between the degrees of color development of HbA1c and hemoglobin other than HbA1c.

Specifically, for example, the developed color signals of HbA1c and hemoglobin other than HbA1c are compared, and in the case where the developed color signal of HbA1c is higher than that of hemoglobin other than HbA1c, a positive determination can be made. On the other hand, in the case where the developed color signal of HbA1c is equal to or lower than that of hemoglobin other than HbA1c, a negative determination can be made.

The immunochromatographic analysis kit of the present invention includes the immunochromatographic analysis device of the present invention, which includes the sample addition part 12 containing an anionic surfactant, the labeling substance retaining part 13, the chromatography medium part 14 having a detection part supported thereon, and the absorption part 15, and the analyte dilution solution, which is for diluting and developing an analyte, and contains a nonionic surfactant.

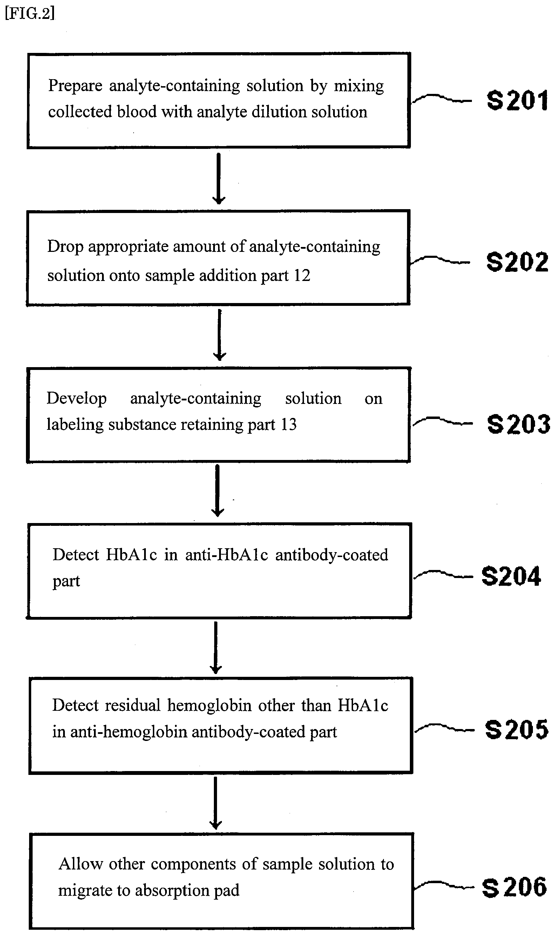

FIG. 2 is a flowchart when measuring the concentration of HbA1c with respect to the amount of total hemoglobin in blood using the immunochromatographic analysis kit of the present invention.

As shown in the flowchart of FIG. 2, first, collected blood and an analyte dilution solution are mixed, whereby an analyte-containing solution is prepared (S201). An appropriate amount of the obtained analyte-containing solution is dropped onto the sample addition part 12 (S202). Subsequently, the analyte-containing solution is developed on the labeling substance retaining part 13 by a capillary phenomenon (S203). The analyte-containing solution developed on the labeling substance retaining part 13 reaches the anti-HbA1c antibody-coated part 16 supported on the chromatography medium part 14, and only HbA1c in the sample solution reacts in the part and is detected (S204).

Subsequently, further the analyte-containing solution developed on the chromatography medium part 14 reaches the anti-hemoglobin antibody-coated part 17, and hemoglobin which did not react in the anti-HbA1c antibody-coated part 16 in the analyte-containing solution reacts and is detected (S205). The other components of the sample solution do not react and migrate to the absorption part 15 (S206). In this manner, hemoglobin other than HbA1c and HbA1c in the sample solution are detected, respectively.

In the present invention, the anionic surfactant to serve as the N-terminal exposure agent is incorporated in the sample addition part 12, and therefore, unlike the conventional art, it is not necessary to prepare two solutions: a sample solution containing an N-terminal exposure agent and a developing solution, nor to drop these two solutions separately onto the sample addition part 12.

Therefore, according to the present invention, it becomes possible to measure various components contained in various analytes such as blood and urine efficiently in a short measurement time without the need for complicated measurement preparation and operations.

### EXAMPLES

Hereinafter, the present invention will be further described by way of Examples and Comparative Examples, however, the present invention is not limited to the following examples.

### Example 1

An immunochromatographic analysis device 1 as shown in FIGS. 1(a) and 1(b) was prepared according to the following procedure.

### (1) Preparation of sample addition part 12

To a glass fiber pad (manufactured by Millipore, Inc., trade name: Glass Fiber Conjugate Pad, size: length 32 mm (in the developing direction of the analyte-containing solution), width 150 mm, thickness 0.43 mm), sodium dodecyl sulfate (SDS) was added uniformly at a rate of of 60 µg/cm², followed by drying at 50°C for 4 hours, whereby a sample addition part 12 was prepared.

### (2) Preparation of anti-HbA1c antibody-coated part 16, anti-hemoglobin antibody-coated part 17, and anti-IgG antibody-coated part 18

As a membrane, a sheet composed of nitrocellulose (manufactured by Millipore, Inc., trade name: HF 12250 mm x 25 mm) was used. An anti-HbA1c monoclonal antibody, an anti-hemoglobin monoclonal antibody, or an anti-IgG monoclonal antibody was diluted to a concentration of 1.0 mg/ml with a 10 mM phosphate buffer solution (pH 7.4) containing 5 mass% sucrose and 5 mass% isopropanol. The diluted solutions (150 µL) were applied to different places with a width of 1 mm on the membrane by an antibody applicator (manufactured by BioDot, Inc.), followed by drying at 50°C for 30 minutes and then drying at room temperature overnight, whereby an anti-HbA1c antibody-coated part 16, an anti-hemoglobin antibody-coated part 17, and an anti-IgG antibody-coated part 18 were provided, respectively, on a chromatography medium part 14.

### (3) Preparation of labeling substance solution

To 0.5 mL of a colloidal gold suspension (manufactured by Tanaka Kikinzoku Kogyo K.K., average particle diameter: 40 nm), 0.1 mL of a solution in which an anti-hemoglobin monoclonal antibody was diluted to a concentration of 0.1 mg/mL with Tris buffer (pH 8.5) was added, and the resulting mixture was left to stand at room temperature for 10 minutes. Then, 0.1 mL of a Tris buffer solution (pH 8.5) containing 0.01 mass% PEG-SH (manufactured by NOF Corporation, trade name: SUNBRIGHT ME-200SH, molecular weight: 20,000) was added thereto (PEG-SH concentration after addition: 0.001 mass%), and the resulting mixture was left to stand at room temperature for 10 minutes. Thereafter, the mixture was thoroughly stirred, and then centrifuged at 8000 x g for 15 minutes. After removing the supernatant, 0.1 mL of a phosphate buffer solution (pH 7.4) containing 1 mass% bovine serum albumin was added thereto, whereby a labeling substance solution was prepared.

### (4) Preparation of Immunochromatographic analysis device 1

A solution obtained by adding 100 µL of a phosphate buffer solution (pH 9.0) containing a 25 mass% aqueous solution of trehalose to 220 µL of the labeling substance solution prepared above was added uniformly to a 8 mm x 100 mm glass fiber pad (manufactured by Millipore, Inc.), followed by drying in a vacuum dryer, whereby a labeling substance retaining part 13 was prepared. Subsequently, on a plastic adhesive sheet 11, the sample addition part 12, the labeling substance retaining part 13 labeled with a labeling substance, and the chromatography medium part 14 prepared above were stuck, and a general-purpose absorption part 15 was further stuck. Then, the resulting material was cut to a width of 5 mm by a cutter, whereby an immunochromatographic analysis device 1 was prepared. At this time, the amount of SDS contained per device was 96 µg.

An analyte-containing solution was prepared by stirring the respective components according to the following formulation.

As a nonionic surfactant, a mixture of Triton X-100 (trade name, manufactured by SIGMA, Inc.) and Tween 20 (trade name, manufactured by Wako Pure Chemical Industries, Ltd.) at a mass ratio of 1:5: 1.2 mass%
As a buffer, a Bicine buffer solution: 50 mM
As an inorganic salt, potassium chloride: 0.6 mass%
As an additive, casein sodium: 2.0 mass%
Balance: water

### Collection of analyte

Blood containing HbA1c at different concentrations was collected by pricking the finger of each of healthy adult males and diabetic male patients.

### Preparation of analyte-containing solution

The blood and the analyte dilution solution were mixed at 1:1,000 (former : latter (volume ratio)), whereby an analyte-containing solution was prepared.

### Implementation of immunochromatographic analysis

To the sample addition part 12 of the immunochromatographic analysis device 1 prepared as described above, 110 µL of the analyte-containing solution was supplied, and after 10 minutes, the developed red color signal in the anti-HbA1c antibody-coated part 16 was visually confirmed. The result is shown in Table 1.

Incidentally, the evaluation criteria in the table are as follows.
-: The development of red color cannot be confirmed.
±: The development of red color can be confirmed but the color is very light.
+: The development of red color can be confirmed.
++: The development of strong red color can be confirmed.
+++: The development of very strong red color can be confirmed.

### Example 2

The procedure of Example 1 was repeated except that the constituent material of the sample addition part 12 was changed from the glass fiber pad in Example 1 to a cellulose fiber pad (manufactured by Asahi Kasei Fibers Corporation, trade name: SR-601). The result is shown in Table 1.

### Example 3

The procedure of Example 1 was repeated except that the constituent material of the sample addition part 12 was changed from the glass fiber pad in Example 1 to a polyethylene terephthalate (PET) pad (manufactured by Asahi Kasei Fibers Corporation, trade name: Bemliese A-01). The result is shown in Table 1.

### Example 4

The procedure of Example 1 was repeated except that the constituent material of the sample addition part 12 was changed from the glass fiber pad in Example 1 to a nitrocellulose membrane (manufactured by Millipore, Inc., trade name: HF 120). The result is shown in Table 1.

### Reference Example 1

The procedure of Example 1 was repeated except that unlike Example 1, a sample addition part free from SDS was used, and 10 µL of a sample solution prepared by adding blood to an extraction solution containing an N-terminal exposure agent having the following composition and leaving the resulting mixture to stand for 2 minutes and 100 µL of a developing solution having the following composition were separately dropped onto the sample addition part 12. The result is shown in Table 1. (Composition of extraction solution) as an anionic surfactant, sodium dodecyl sulfate: 1.0 mass%, as an additive, sodium thiocyanate: 100 mM, as an additive, EDTA: 5 mM, balance: water, (Composition of developing solution) as a nonionic surfactant, a mixture of Triton X-100 (trade name, manufactured by SIGMA, Inc.) and Tween 20 (trade name, manufactured by Wako Pure Chemical Industries, Ltd.) at a mass ratio of 1:5: 1.2 mass%, as a buffer, a Bicine buffer solution: 50 mM, as an inorganic salt, potassium chloride: 0.6 mass%, as an additive, casein sodium: 2.0 mass%, balance: water

### Reference Example 2

The procedure of Reference Example 1 was repeated except that unlike Reference Example 1, blood was added to the developing solution without using the extraction solution. The result is shown in Table 1.

### Reference Examples 3 to 5

The procedure of Reference Example 1 was repeated except that unlike Reference Example 1, SDS at a concentration shown in Table 1 and blood were added to the developing solution without using the extraction solution. The results are shown in Table 1.

[Table 1]

**Table 1**

| | Concentration of HbA1c | | |
|---|---|---|---|
| | 5.5% | 7.0% | 9.0% |
| Example 1 (glass fiber pad + SDS) | + | ++ | +++ |
| Example 2 (cellulose fiber pad + SDS) | ± | + | ++ |
| Example 3 (PET pad + SDS) | ± | ± | + |
| Example 4 (nitrocellulose membrane + SDS) | ± | + | + |
| Reference Example 1 (extraction solution + developing solution) | + | ++ | +++ |
| Reference Example 2 (only developing solution) | - | - | ± |
| Reference Example 3 (developing solution + 0.1% SDS) | - | - | + |
| Reference Example 4 (developing solution + 0.2% SDS) | - | ± | + |
| Reference Example 5 (developing solution + 0.4% SDS) | - | - | ± |

### Examples 5 to 8

The procedure of Example 1 was repeated except that the content per device of SDS to be added to the sample addition part 12 and the content of the nonionic surfactant to be contained in the analyte dilution solution in Example 1 were changed to the values shown in Table 2. The results are shown in Table 2.

[Table 2]

**Table 2**

| | Anionic surfactant (µg) | Nonionic surfactant (mass%) | Concentration of HbA1c | | |
|---|---|---|---|---|---|
| | | | 5.5% | 7.0% | 9.0% |
| Example 5 | 480 | 1.2 | + | ++ | +++ |
| Example 6 | 16 | 1.2 | ± | + | ++ |
| Example 7 | 96 | 0.3 | + | + | ++ |
| Example 8 | 96 | 3.0 | + | ++ | +++ |

The results shown in Table 1 and Table 2 reveal that by using the immunochromatographic analysis device of the present invention, unlike Reference Example 1 of the conventional art, two solutions: a sample solution and a developing solution are not needed, and it is only necessary to drop one solution: an analyte-containing solution onto the sample addition part, and it is possible to measure various components contained in various analytes such as blood and urine efficiently in a short measurement time equivalently to Reference Example 1 of the conventional art without the need for complicated measurement preparation and operations.

Reference Example 2 is an example in which the sample addition part free from SDS was used, and only the developing solution was used, and therefore, although the concentration of HbA1c was high, the degree of color development was poor.

Reference Examples 3 to 5 are examples in which the sample addition part free from SDS was used, and SDS was added to the developing solution, however, although the degree of color development was improved as compared with Reference Example 2, a satisfactory level could not yet be reached.

While the present invention has been described in detail with reference to specific embodiments, it is apparent to those skilled in the art that various changes and modifications can be made without departing from the spirit and scope of the present invention. The present application is based on Japanese Patent Application (Japanese Patent Application No. 2014-091510) filed on April 25, 2014 and the entire contents of which are incorporated herein by reference.

### REFERENCE SIGNS LIST

- 1: immunochromatographic analysis device
- 11: plastic adhesive sheet
- 12: sample addition part
- 13: labeling substance retaining part
- 14: chromatography medium part
- 15: absorption part
- 16: anti-HbA1c antibody-coated part coated with anti-HbA1c antibody
- 17: anti-hemoglobin antibody-coated part coated with anti-hemoglobin antibody
- 18: anti-IgG antibody-coated part coated with anti-IgG antibody

## Claims

1. An immunochromatographic analysis device for developing an analyte-containing solution obtained by diluting an analyte containing a detection target with an analyte dilution solution, comprising a sample addition part, a labeling substance retaining part, a chromatography medium part having a detection part supported thereon, and an absorption part, wherein
the sample addition part contains an anionic surfactant.

2. The immunochromatographic analysis device according to claim 1, wherein the sample addition part is one member selected from the group consisting of glass fiber, cellulose, and polyethylene terephthalate.

3. The immunochromatographic analysis device according to claim 1 or 2, wherein the sample addition part contains the anionic surfactant in an amount of 8 to 800 µg.

4. The immunochromatographic analysis device according to any one of claims 1 to 3, wherein the detection target is a glycated protein in blood.

5. The immunochromatographic analysis device according to claim 4, wherein the glycated protein in blood is HbA1c.

6. The immunochromatographic analysis device according to any one of claims 1 to 5, wherein the anionic surfactant is sodium dodecyl sulfate (SDS).

7. An immunochromatographic analysis method for detecting a detection target contained in an analyte using an immunochromatographic analysis device which includes a sample addition part containing an anionic surfactant, a labeling substance retaining part, a chromatography medium part having a detection part supported thereon, and an absorption part, comprising the following steps (1) to (4):
(1) a step of adding an analyte-containing solution obtained by diluting an analyte with an analyte dilution solution to the sample addition part;
(2) a step of recognizing the detection target by a labeling substance retained in the labeling substance retaining part;
(3) a step of developing the analyte and the labeling substance in the chromatography medium part as a mobile phase; and
(4) a step of detecting the detection target in the developed mobile phase in the detection part.

8. The method according to claim 7, wherein the analyte dilution solution contains a nonionic surfactant.

9. The method according to claim 8, wherein the analyte dilution solution contains the nonionic surfactant in an amount of 0.01 to 5 mass%.

10. The method according to any one of claims 7 to 9, wherein the sample addition part contains the anionic surfactant in an amount of 8 to 800 µg.

11. An immunochromatographic analysis kit, comprising an immunochromatographic analysis device, which includes a sample addition part, a labeling substance retaining part, a chromatography medium part having a detection part supported thereon, and an absorption part, and an analyte dilution solution for diluting and developing an analyte, wherein the analyte dilution solution contains a nonionic surfactant, and the sample addition part contains an anionic surfactant.
